(19) Europäisches Patentamt
European Patent Office
Office européen des brevets

(11) Veröffentlichungsnummer: **0 321 849 B1**

(12) **EUROPÄISCHE PATENTSCHRIFT**

(45) Veröffentlichungstag der Patentschrift: **21.07.93**

(51) Int. Cl.[5]: **C12N 9/10**, C12P 13/04, C12P 17/18

(21) Anmeldenummer: **88120945.6**

(22) Anmeldetag: **15.12.88**

(54) **Verfahren zur enzymatischen Hydrolyse von alpha-Aminoadipinyl-Monoaminoverbindungen.**

(30) Priorität: **21.12.87 DE 3743323**

(43) Veröffentlichungstag der Anmeldung:
**28.06.89 Patentblatt 89/26**

(45) Bekanntmachung des Hinweises auf die Patenterteilung:
**21.07.93 Patentblatt 93/29**

(84) Benannte Vertragsstaaten:
**AT BE CH DE ES FR GB GR IT LI LU NL SE**

(56) Entgegenhaltungen:
**EP-A- 0 275 901**
**US-A- 4 774 179**

(73) Patentinhaber: **HOECHST AKTIENGESELLSCHAFT**
**Postfach 80 03 20**
**W-6230 Frankfurt am Main 80(DE)**

(72) Erfinder: **Aretz, Werner, Dr.**
**Am Wäldchen 13**
**W-6240 Königstein/Taunus(DE)**
Erfinder: **Sauber, Klaus, Dr.**
**Friedrich-Ebert-Strasse 15**
**W-6231 Schwalbach/Taunus(DE)**

**EP 0 321 849 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

γ-Glutamyl-Transpeptidasen (im folgenden γ-GTP) spielen in tierischen Geweben und in Mikroorganismen eine wichtige Rolle im Aminosäurestoffwechsel und im Glutathion-Zyklus [Meth. Enzymol. 77, 237 (1981)]. Sie sind für den Transport verschiedener Aminosäuren in Form ihrer γ-Glutamyl-Derivate, die Bildung von Polyglutaminsäure in Bacilli sowie den Abbau von Glutathion (γ-Glutamyl-Cysteinyl-Glycin) verantwortlich.

Es wurde bereits vorgeschlagen (EP 0 275 901), γ-GTP zur Hydrolyse von Adipinyl- oder Glutaryl-Monoaminoverbindungen zu verwenden.

Es wurde nun überraschend gefunden, daß γ-GTP die Hydrolyse von α-Aminoadipinyl-Monoaminoverbindungen der Formel I

$$HOOC-\underset{NH_2}{\underset{|}{CH}}-(CH_2)_3-\underset{O}{\underset{\|}{C}}-NH-R^1 \qquad (I)$$

worin

$R^1$ Aminosäuren, Dipeptide, Cepheme, Cephame oder deren Derivate bedeutet,

katalysiert.

Dies ist um so überraschender, als bisher angenommen wurde, daß weder $C_4$- noch $C_6$-Seitenketten vom aktiven Zentrum der γ-GTP akzeptiert werden (siehe Agric. Biol. Chem. 42, 1978, S. 371 - 81).

Die Erfindung betrifft somit:

1. Eine γ-Glutamyltranspeptidase gekennzeichnet durch
   - ein Molekulargewicht von 40 000 bis 80 000,
   - einen isoelektrischen Punkt bei pH 4,4 bis 5,9,
   - für L-γ-Glutamylparanitroanilid als Substrat ein pH-Optimum in dem Bereich 6,5 bis 10 sowie einen Km-Wert von 9 bis 36 µM bei pH 8 und
   - die Hydrolyse von α-Aminoadipinyl-Monoaminoverbindungen der Formel I,

$$HOOC-\underset{NH_2}{\underset{|}{CH}}-(CH_2)_3-\underset{O}{\underset{\|}{C}}-NH-R^1 \qquad (I)$$

   in der

   $R^1$ Aminosäuren, Dipeptide, Cepheme, Cephame oder deren Derivate bedeutet.

2. Ein Verfahren zur Herstellung der unter 1. charakterisierten γ-Glutamyltranspeptidase, das dadurch gekennzeichnet ist, daß Bakterien der Gattungen Pseudomonas, Proteus, Arthrobacter und Bacillus in einem Nährmedium kultiviert werden, bis sich das genannte γ-GTP in dem Nährmedium anhäuft.
3. Die Verwendung der unter 1. charakterisierten γ-Glutamyltranspeptidase zur Hydrolyse von α-Aminoadipinyl-Monoaminoverbindungen der Formel I.

Im folgenden wird die Erfindung detailliert beschrieben, insbesondere in ihren bevorzugten Ausführungsformen. Weiterhin wird die Erfindung in den Patentansprüchen definiert.

Die γ-Glutamyltranspeptidase (γ-GTP) katalysiert die Hydrolyse von α-Aminoadipinyl-Monoaminoverbindungen der Formel I, wie oben definiert, zu der entsprechenden Säure und der Monoaminoverbindung. Es werden bevorzugt 7-Aminocephalosporansäure-Derivate als Substrat eingesetzt.

Das Enzym kommt in Mikroorganismen periplasmatisch und extrazellulär vor und läßt sich charakterisieren durch ein Molekulargewicht von 40.000 bis 80.000, bevorzugt 50.000 bis 70.000, inbesondere 55.000 bis 65.000 sowie durch einen isoelektrischen Punkt, der bei einem pH-Wert von 4,4 bis 5,9, bevorzugt 4,8 bis 5,5, liegt. Das pH-Optimum für L-γ-Glutamylparanitroanilid als Substrat liegt in dem pH-Bereich 6,5 bis 10. Für das gleiche Substrat hat die erfindungsgemäße Transpeptidase einen Km-Wert von 9 bis 36 µM, bevorzugt 15 bis 20 µM, insbesondere 17,8 µM, bei pH 8.

In Gegenwart von Azaserin oder Jodacetamid wird die erfindungsgemäße γ-GTP irreversibel gehemmt. Bei Anwesenheit von Kupfer, Quecksilber und einer Mischung von Serin und Borat sowie in Gegenwart von 7-Aminocephalosporansäure zeigt das Enzym eine reversible Hemmung.

Die Herstellung der γ-GTP erfolgt mit Hilfe von Mikroorganismen wie auch in der Europäischen Patentanmeldung EP 0 275 901 beschrieben. Bei diesem Verfahren werden Bakterien, insbesondere die Gattungen Pseudomonas, Proteus, Arthrobacter und Bacillus in einem Nährmedium kultiviert, bis sich γ-GTP in dem Nährmedium anhäuft. Geeignet sind beispielsweise: Pseudomonas putida ATCC 17390, Pseudomonas aeruginosa NCTC 10701, Proteus vulgaris ATCC 9634, Arthrobacter parafineus ATCC 31917 sowie Pseudomonas fragi DSM 3881 und Bacillus subtilis IFO 3025. Insbesondere bevorzugt wird das Enzym aus Bac. subtilis IFO 3025 gewonnen. Auch Mutanten und Varianten der genannten Mikroorganismen sind geeignet.

Die Anzucht der Mikroorganismen erfolgt aerob einzeln oder in Mischkultur beispielsweise submers unter Schütteln oder Rühren in Schüttelkolben oder Fermentern, gegebenenfalls unter Einführung von Luft oder Sauerstoff. Die Fermentation kann in einem Temperaturbereich von etwa 20 bis 37 °C, vorzugsweise bei etwa 25 bis 30 °C, insbesondere bei 28 bis 30 °C, erfolgen. Es wird in einem pH-Bereich zwischen 5 und 8,5, vorzugsweise zwischen 5,5 und 8,0, fermentiert. Unter diesen Bedingungen zeigt die Kulturbrühe im allgemeinen nach 1 bis 3 Tagen eine nennenswerte Akkumulation des Enzyms. Die Synthese der γ-GTP beginnt in der späten log-Phase und erreicht ihr Maximum in der stationären Wachstumsphase. Die Produktion des periplasmatischen Enzyms kann mit Hilfe von Aktivitätstests durch HPLC-Analyse bzw. photometrisch verfolgt werden.

Die zur Produktion des γ-GTP verwendete Nährlösung enthält 0,2 bis 5 %, bevorzugt 0,5 bis 2 %, organische Stickstoffverbindungen sowie anorganische Salze. Als organische Stickstoffverbindungen kommen in Betracht: Aminosäuren, Peptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte. An anorganischen Salzen kann die Nährlösung beispielsweise Chloride, Carbonate, Sulfate oder Phosphate der Alkali-oder Erdalkalimetalle, Eisen, Zink und Mangan enthalten, aber auch Ammoniumsalze und Nitrate.

Der Zusatz von assimilierbaren Kohlenhydraten steigert die Biomasseausbeute. Kohlenhydrate werden ebenfalls in den obengenannten Konzentrationen zugegeben. Als bevorzugte Kohlenstoffquelle können beispielsweise Zucker, wie Glucose oder Saccharose, sowie kohlenhydrathaltige Naturprodukte, wie Malzextrakt, der Nährlösung zugegeben werden.

Die optimalen Fermentations-Bedingungen sind für jeden Mikroorganismus zwar unterschiedlich, aber entweder dem Fachmann schon bekannt oder aber in leichten Vorversuchen festzustellen.

Die Reinigung kann nach klassischen Verfahren über Lysozym-Aufschluß, Ammoniumsulfat-Fällung, Ionenaustauscher- und Gelpermeationschromatographie erfolgen. Die Kopplung des Enzyms ist nach gängigen Methoden möglich (Colowick und Kaplan, Meth. Enzymol., Vol. XLIV).

Für die enzymatische Umsetzung können sowohl ganze Zellen in freier oder immobilisierter Form unter Zusatz von ß-Laktamase-Inaktivatoren, beispielsweise Clavulansäure oder Thienamycin, als auch das isolierte Enzym , das ebenfalls trägergebunden sein kann, eingesetzt werden. Geeignete Materialien für die Immobilisierung ganzer Zellen sind beispielsweise Chitosan, Alginat, χ-Carrageenan, Polyacrylhydrazide und weitere bekannte Stoffe aus literaturbekannten Verfahren (K. Venkatsubramanian, Immob. Cells (1979), ACS Symposium Series, S. 106).

Die Hydrolyse-Reaktion wird am günstigsten bei ca. pH 6,6 bis 8 sowie bei einer Temperatur von ca. 28 bis 38 °C durchgeführt. Bevorzugt wird die Verbindung der Formel I, in der $R^1$ den Rest

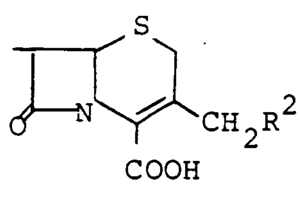

bedeutet, in dem $R^2$ Wasserstoff, OH oder

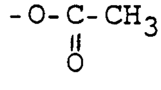

ist.

Die γ-GTP hat insbesondere für die Gewinnung von 7-Aminocephalosporansäure aus Cephalosporin C technische Bedeutung. Bisher wurde jedoch zunächst immer mit Hilfe einer Hefe (Trigonopsis variabilis) aus Cephalosporin C die Glutaryl-7-aminocephalosporansäure erzeugt, welche dann erst in einem zweiten Reaktionsschritt enzymatisch zur 7-Aminocephalosporansäure hydrolysiert werden konnte.

Mit dem erfindungsgemäßen Verfahren ist es nunmehr möglich, in einem einzigen Schritt aus Cephalosporin C die 7-Aminocephalosporansäure herzustellen.

In den sich anschließenden Beispielen wird die Erfindung noch weitergehend beschrieben. Prozentangaben beziehen sich auf das Gewicht, wenn nicht anders angegeben.

**Beispiel 1**

Die Stammhaltung der γ-GTP-produzierenden Mikroorganismen erfolgt auf Schrägagar folgender Zusammensetzung:

| | |
|---|---|
| Glucose | 1 % |
| Caseinpepton | 0,4 % |
| Fleischextrakt | 0,4 % |
| Hefeextract | 0,05 % |
| Leberextract | 0,05 % |
| NaCl | 0,25 % |
| pH 7,2 | |

Die Schrägröhrchen werden 2 Tage bei 28 °C bebrütet. Anschließend werden die Zellen mit 10 ml physiologischer Kochsalzlösung abgeschwemmt und 1 ml dieser Suspension zum Animpfen von 50 ml Vorkultur folgender Zusammensetzung in einem Erlenmeyerkolben mit 300 ml Fassungsvermögen verwendet:

| | |
|---|---|
| Pepton | 1 % |
| Malzextrakt | 0,5 % |
| pH 7,0 | |

Der Kolben wird bei 190 Upm auf einem Rotationsschüttler 24 Stunden bei 30 °C inkubiert. 2,5 ml dieser Kultur dienen als Inokulum von 50 ml Hauptkultur:

| Bacilli | |
|---|---|
| Pepton | 0.12 % |
| Hefeextrakt | 0,12 % |
| Glucose | 0,25 % |
| Na-Lactat (60 %) | 5,6 ml |
| $NH_4Cl$ | 0,12 % |
| $K_2HPO_4$ | 0,12 % |
| $KH_2PO_4$ | 0,034 % |
| $MgSO_4$ x 7 $H_2O$ | 0,025 % |
| NaCl | 0,5 % |
| KCl | 0,5 % |
| $CaCl_2$ x 2 $H_2O$ | 0,0015 % |
| $MnCl_2$ x 4 $H_2O$ | 0,0007 % |
| $Fe(NH_4)$Citrat | 0,00015 % |

Die Kultur wird 24 Stunden bei 28 °C und einer Schüttelfrequenz von 190 Upm inkubiert und anschließend durch Zentrifugation abgeerntet.

In der folgenden Tabelle sind $\gamma$-GTP-Aktivitäten einiger Stämme aufgeführt:

| Stamm | $\gamma$-GTP (mU/ml Kulturlösung) |
|---|---|
| B. subtilis IFO 3025 | 60 |
| " " IFO 3013 | 15 |
| " " IFO 3335 | 25 |

**Beispiel 2**

Analog Beispiel 1 wird eine Vorkultur mit Bac. subtilis IFO 3025 angezogen. 50 ml dieser Kultur dienen als Inokulum für 2 l Hauptkulturlösung in einem 5 l-Fermenter. Die Anzucht des Stammes erfolgt bei 34 °C und einem Sauerstoffpartialdruck von 70 %. Die Bildung der $\gamma$-GTP wird photometrisch verfolgt und die Kultur bei maximalem Enzymtiter abgeerntet. Unter den gegebenen Bedingungen wird ein $\gamma$-GTP-Titer von 150 mU/ml Kulturlösung erreicht.

**Beispiel 3**

9 l Kulturlösung werden mittels Cross-Flow-Filtration (Ausschlußgrenze 300 000 Dalton) in Kulturfiltrat und Zellmasse getrennt. Das so gewonnene Kulturfiltrat enthält 1350 U $\gamma$-GTP-Aktivität.

Durch Zugabe von Ammoniumsulfat ad 70 % d. S. wird das Enzym gefällt und in einem 1/10 des Volumens wieder aufgenommen. Nach Dialyse gegen 20 mM Tris, pH 8,0, wird über eine DEAE-Cellulose Säule (DE 52, Whatman) das Enzym weiter gereinigt. Die aktiven Eluate werden vereinigt und konzentriert. Ein so gewonnenes $\gamma$-GTP-Präparat (mit ca. 25 U $\gamma$-GTP/ml) wird für die Umsetzungen verwendet.

**Beispiel 4**

Für eine präparative Umsetzung von Desacetyl-CPC wird folgender Ansatz gewählt:
100 $\mu$l Enzymkonzentrat, hergestellt nach Beispiel 3, und
100 $\mu$l 40 mM Desacetyl-CPC, gelöst in 20 mM Kaliumphosphatpuffer, pH 7,3 werden bei einer Temperatur von 33 °C inkubiert.

Unter den gewählten Bedingungen entstehen bis zu 16 % Desacetyl-7-Aminocephalosporansäure.

**Beispiel 5**

Analog den in Beispiel 4 angeführten Inkubationsbedingungen werden 3 % 7-Aminocephalosporansäure aus CPC freigesetzt.

**Beispiel 6**

Aktivitätsbestimmung von $\gamma$-GTP

a) HPLC-Test

50 $\mu$l 80 mM Desacetyl-CPC werden mit 100 bis 140 $\mu$l 250 mM Kaliumphosphatpuffer, pH 5,0, und 10 bis 50 $\mu$l Enzymlösung vermischt und bei 33 °C inkubiert. Nach jeweils 10 Minuten werden 20 $\mu$l Probe entnommen. Die Reaktion wird mit 20 $\mu$l Methanol abgestoppt. Es wird zentrifugiert und im Verhältnis 1 : 10 mit Wasser verdünnt. Eine Probe von 10 $\mu$l wird mittels HPLC auf den Gehalt an 7-Aminocephalosporansäure untersucht.

| | |
|---|---|
| Stationäre Phase: | C-18-Kieselgel |
| Mobile Phase: | $KH_2PO_4$ 50 mM in $H_2O$/ MeOH (80 : 20) + 0,001 % Tetrabutylammoniumsulfat |

b) Photometrischer Test

600 µl L-γ-Glutamyl-p-nitroanilid (166 µM)
300 µl Kaliumphosphatpuffer, pH 5,7, 50 mM und
100 µl Kulturlösung werden miteinander vermischt und bei 37 °C inkubiert.

$$\epsilon_{405} = 9620 \frac{1}{Mol \cdot cm}$$

**Patentansprüche**
**Patentansprüche für folgende Vertragsstaaten : AT, BE, CH, DE, FR, GB, GR, IT, LI, LU, NL, SE**

**1.** γ-Glutamyltranspeptidase gekennzeichnet durch
- ein Molekulargewicht von 40 000 bis 80 000,
- einen isoelektrischen Punkt bei PH 4,4 bis 5,9,
- für L-γ-Glutamylparanitroanilid als Substrat ein pH-Optimum in dem Bereich 6,5 bis 10 sowie einen Km-Wert von 9 bis 36 µM bei pH 8 und
- die Hydrolyse von α-Aminoadipinyl-Monoaminoverbindungen der Formel I,

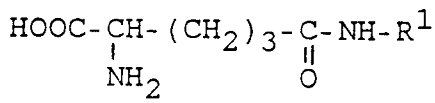 (I)

in der
$R^1$ Aminosäuren, Dipeptide, Cepheme, Cephame oder deren Derivate bedeutet.

**2.** Verfahren zur Herstellung der γ-Glutamyltranspeptidase nach Anspruch 1, dadurch gekennzeichnet, daß Bakterien der Gattungen Pseudomonas, Proteus, Arthrobacter und Bacillus in einem Nährmedium kultiviert werden, bis sich γ-GTP nach Anspruch 1 in dem Nährmedium anhäuft.

**3.** Verfahren nach Anspruch 2, dadurch gekennzeichnet, daß die γ-Glutamyltranspeptidase durch Kultivierung von B. subtilis IFO 3025 erhalten wird.

**4.** Verwendung der γ-Glutamyltranspeptidase nach Anspruch 1 zur Hydrolyse von α-Aminoadipinyl-Monoaminoverbindungen der Formel I,

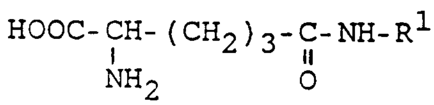 (I)

in der
$R^1$ Aminosäuren, Dipeptide, Cepheme, Cephame oder deren Derivate bedeutet.

**5.** Verwendung nach Anspruch 4, dadurch gekennzeichnet, daß die Verbindung der Formel I hydrolysiert wird, in der $R^1$ den Rest

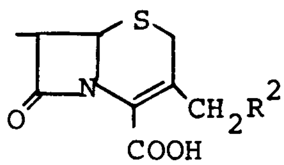

bedeutet, in dem $R^2$ Wasserstoff, OH oder

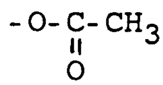

ist

6. Verwendung nach Anspruch 4 oder 5, dadurch gekennzeichnet, daß die Hydrolyse bei einem pH-Wert von 6,6 bis 8,0 durchgeführt wird.

7. Verwendung nach einem oder mehreren der Ansprüche 4 bis 6, dadurch gekennzeichnet, daß die Hydrolyse bei 28 bis 38 °C durchgeführt wird.

**Patentansprüche für folgenden Vertragsstaat : ES**

1. Verfahren zur Herstellung von γ-Glutamyltranspeptidase mit
   - einem Molekulargewicht von 40 000 bis 80 000,
   - einem isoelektrischen Punkt bei pH 4,4 bis 5,9,
   - für L-γ-Glutamylparanitroanilid als Substrat ein pH-Optimum in dem Bereich 6,5 bis 10 sowie einem Km-Wert von 9 bis 36 µM bei pH 8,

   die die Hydrolyse von α-Aminoadipinyl-Monoaminoverbindungen der Formel I

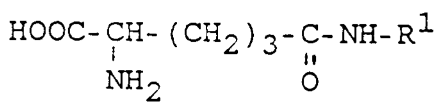
(I)

   in der
   $R^1$ Aminosäuren, Dipeptide, Cepheme, Cephame oder deren Derivate bedeutet, bewirkt, dadurch gekennzeichnet, daß Bakterien der Gattungen Pseudomonas, Proteus, Arthrobacter und Bacillus in einem Nährmedium kultiviert werden, bis sich das genannte γ-GTP in dem Nährmedium anhäuft.

2. Verfahren nach Anspruch 1, dadurch gekennzeichnet, daß die γ-Glutamyltranspeptidase durch Kultivierung von B. subtilisIFO 3025 erhalten wird.

3. Verwendung der γ-Glutamyltranspeptidase erhältlich nach Anspruch 2 zur Hydrolyse von α-Aminoadipinyl-Monoaminoverbindungen der Formel I

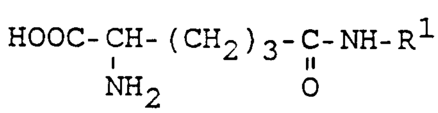
(I)

   in der
   $R^1$ Aminosäuren, Dipeptide Cepheme, Cephame oder deren Derivate bedeutet.

**4.** Verwendung nach Anspruch 3, dadurch gekennzeichnet, daß die Verbindung der Formel I hydrolysiert wird, in der $R^1$ den Rest

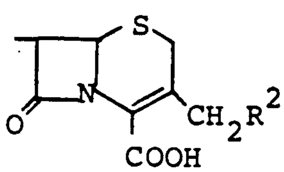

bedeutet, in dem $R^2$ Wasserstoff, OH oder

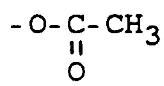

ist

**5.** Verwendung nach Anspruch 3 oder 4, dadurch gekennzeichnet, daß die Hydrolyse bei einem pH-Wert von 6,6 bis 8,0 durchgeführt wird.

**6.** Verwendung nach einem oder mehreren der Ansprüche 3 bis 5, dadurch gekennzeichnet, daß die Hydrolyse bei 28 bis 38 °C durchgeführt wird.

**Claims**

**Claims for the following Contracting States : AT, BE, CH, DE, FR, GB, GR, IT, LI, LU, NL, SE**

**1.** $\gamma$-Glutamyltranspeptidase having the following properties
- a molecular weight of 40,000 to 80,000
- an isoelectric point at pH 4.4 to 5.9,
- for L-$\gamma$-glutamylparanitroanilide as substrate a pH optimum in the range 6.5 to 10, and a Km of 9 to 36 $\mu$m at pH 8, and
- hydrolysis of $\alpha$-aminoadipinyl-monoamino compounds of the formula I

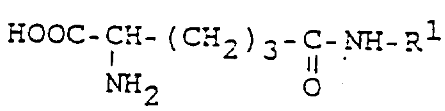

(I)

in which
$R^1$ denotes amino acids, dipeptides, cephems, cephams or derivatives thereof.

**2.** A process for the preparation of the $\gamma$-glutamyltranspeptidase as claimed in claim 1, which comprises cultivation of bacteria of the genera Pseudomonas, Proteus, Arthrobacter and Bacillus in a nutrient medium until $\gamma$-GTP as claimed in claim 1 accumulates in the nutrient medium.

**3.** The process as claimed in claim 2, wherein the $\gamma$-glutamyltranspeptidase is obtained by cultivation of B. subtilis IFO 3025.

**4.** The use of the $\gamma$-glutamyltranspeptidase as claimed in claim 1 for the hydrolysis of $\alpha$-aminoadipinyl-monoamino compounds of the formula I

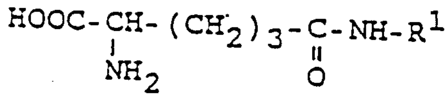

(I)

in which
$R^1$ denotes amino acids, dipeptides, cephems, cephams or derivatives thereof.

**5.** The use as claimed in claim 4, wherein the compound of the formula I in which $R^1$ denotes the radical

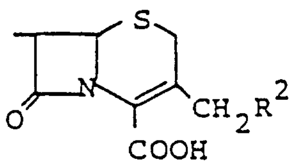

in which $R^2$ is hydrogen, OH or

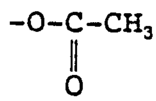

is hydrolyzed.

**6.** The use as claimed in claim 4 or 5, wherein the hydrolysis is carried out at a pH of 6.6 to 8.0.

**7.** The use as claimed in one or more of claims 4 to 6, wherein the hydrolysis is carried out at 28 to 38 ° C.

**Claims for the following Contracting State : ES**

**1.** A process for the preparation of γ-glutamyltranspeptidase with
- a molecular weight of 40,000 to 80,000
- an isoelectric point at pH 4.4 to 5.9,
- for L-γ-glutamylparanitroanilide as substrate a pH optimum in the range 6.5 to 10, and a Km of 9 to 36 μm at pH 8,
- which effects the hydrolysis of α-aminoadipinyl-monoamino compounds of the formula I

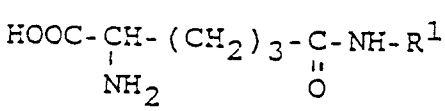

(I)

in which
$R^1$ denotes amino acids, dipeptides, cephems, cephams or derivatives thereof, which comprises cultivation of bacteria of the genera Pseudomonas, Proteus, Arthrobacter and Bacillus in a nutrient medium until the said γ-GTP accumulates in the nutrient medium.

**2.** The process as claimed in claim 1, wherein the γ-glutamyltranspeptidase is obtained by cultivation of B. subtilis IFO 3025.

3. The use of the γ-glutamyltranspeptidase obtainable as claimed in claim 2 for the hydrolysis of α-aminoadipinyl-monoamino compounds of the formula I

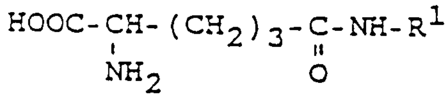 (I)

in which
$R^1$ denotes amino acids, dipeptides, cephems, cephams or derivatives thereof.

4. The use as claimed in claim 3, wherein the compound of the formula I in which $R^1$ denotes the radical

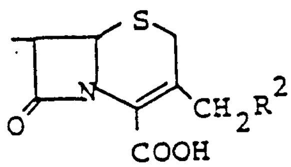

in which $R^2$ is hydrogen, OH or

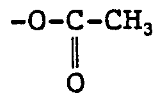

is hydrolyzed.

5. The use as claimed in claim 3 or 4, wherein the hydrolysis is carried out at a pH of 6.6 to 8.0.

6. The use as claimed in one or more of claims 3 to 5, wherein the hydrolysis is carried out at 28 to 38 °C.

**Revendications**

**Revendications Pour les Etats Contractants Suivants : AT, BE, CH, DE, FR, GB, GR, IT, LI, LU, NL, SE**

1. γ-glutamyltranspeptidase caractérisée par
   - une masse moléculaire de 40 000 à 80 000,
   - un point isoélectrique à pH de 4,4 à 5,9,
   - pour le L-γ-glutamyl-p-nitroanilide en tant que substrat, un pH optimum dans l'intervalle allant de 6,5 à 10, ainsi qu'une valeur $K_m$ allant de 9 à 36 µM à pH 8, et
   - l'hydrolyse de composés α-aminoadipinyl-monoaminés de formule I

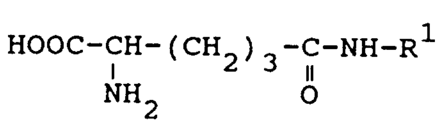 (I)

dans laquelle $R^1$ représente des aminoacides, dipeptides, céphèmes, céphams ou leurs dérivés.

2. Procédé pour la production de la γ-glutamyltranspeptidase selon la revendication 1, caractérisé en ce que l'on cultive dans un milieu nutritif des bactéries appartenant aux genres *Pseudomonas, Proteus,*

*Arthrobacter* et *Bacillus*, jusqu'à ce que de la γ-GTP selon la revendication 1 s'accumule dans le milieu nutritif.

**3.** Procédé selon la revendication 2, caractérisé en ce que l'on obtient la γ-glutamyltranspeptidase par culture de *B. subtilis* IFO 3025.

**4.** Utilisation de la γ-glutamyltranspeptidase selon la revendication 1, pour l'hydrolyse de composés α-aminoadipinyl-monoaminés de formule I

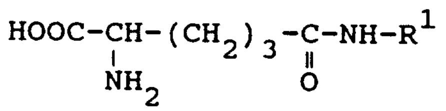
(I)

dans laquelle $R^1$ représente des aminoacides, dipeptides, céphèmes, céphams ou leurs dérivés.

**5.** Utilisation selon la revendication 4, caractérisée en ce que l'on hydrolyse le composé de formule I dans lequel $R^1$ représente le radical

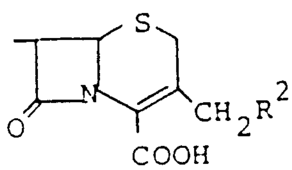

dans lequel $R^2$ est un atome d'hydrogène, OH ou

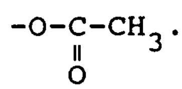

**6.** Utilisation selon la revendication 4 ou 5, caractérisée en ce que l'on effectue l'hydrolyse à un pH de 6,6 à 8,0.

**7.** Utilisation selon une ou plusieurs des revendications 4 à 6, caractérisée en ce que l'on effectue l'hydrolyse à 28-38 °C.

**Revendications Pour l'Etat Contractant suivant : ES**

**1.** Procédé pour la production de γ-glutamyltranspeptidase, ayant
- une masse moléculaire de 40 000 à 80 000,
- un point isoélectrique à pH de 4,4 à 5,9,
- pour le L-γ-glutamyl-p-nitroanilide en tant que substrat, un pH optimum dans l'intervalle allant de 6,5 à 10, ainsi qu'une valeur $K_m$ allant de 9 à 36 μM à pH 8,

qui provoque l'hydrolyse de composés α-aminoadipinyl-monoaminés de formule I

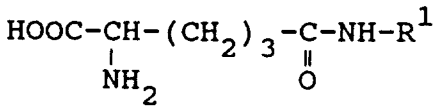
(I)

dans laquelle $R^1$ représente des aminoacides, dipeptides, céphèmes, céphams ou leurs dérivés, caractérisé en ce que l'on cultive dans un milieu nutritif des bactéries appartenant aux genres *Pseudomonas, Proteus, Arthrobacter* et *Bacillus,* jusqu'à ce que ladite γ-GTP s'accumule dans le

milieu nutritif.

2. Procédé selon la revendication 1, caractérisé en ce que l'on obtient la $\gamma$-glutamyltranspeptidase par culture de *B. subtilis* IFO 3025.

3. Utilisation de la $\gamma$-glutamyltranspeptidase pouvant être obtenue selon la revendication 2, pour l'hydrolyse de composés $\alpha$-aminoadipinyl-monoaminés de formule I

$$HOOC-\underset{\substack{| \\ NH_2}}{CH}-(CH_2)_3-\underset{\substack{\| \\ O}}{C}-NH-R^1 \qquad (I)$$

dans laquelle $R^1$ représente des aminoacides, dipeptides, céphèmes, céphams ou leurs dérivés.

4. Utilisation selon la revendication 3, caractérisée en ce que l'on hydrolyse le composé de formule I dans lequel $R^1$ représente le radical

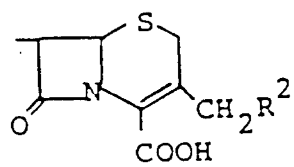

dans lequel $R^2$ est un atome d'hydrogène, OH ou

$$-O-\underset{\substack{\| \\ O}}{C}-CH_3 .$$

5. Utilisation selon la revendication 3 ou 4, caractérisée en ce que l'on effectue l'hydrolyse à un pH de 6,6 à 8,0.

6. Utilisation selon une ou plusieurs des revendications 3 à 5, caractérisée en ce que l'on effectue l'hydrolyse à 28-38 °C.